# EUROPEAN PATENT APPLICATION

(11) **EP 3 424 943 A1**
(43) Date of publication of application: **09.01.2019**
(21) Application number: 17760002.0
(22) Date of filing: 28.02.2017
(51) Int. Cl.: C07K 14/47, A23J 3/08, A23L 33/18, A61K 38/08, A61K 38/17, A61P 3/04, C07K 1/12, C07K 16/04

(54) **PEPTIDE**

(30) Priority: 29.02.2016 JP 2016037673
(71) Applicant: Kyoto University, Kyoto-shi, Kyoto 606-8501 (JP); Kazusa DNA Research Institute, Kisarazu-shi, Chiba 292-0818 (JP)
(72) Inventor: OHINATA, Kousaku, Kyoto-shi Kyoto 606-8501 (JP); NAKATO, Junya, Kyoto-shi Kyoto 606-8501 (JP); AOKI, Hayato, Kyoto-shi Kyoto 606-8501 (JP); IWAKURA, Hiroshi, Kyoto-shi Kyoto 606-8501 (JP); SUZUKI, Hideyuki, Kisarazu-shi Chiba 292-0818 (JP)
(74) Representative: Winter, Brandl, Fürniss, Hübner, Röss, Kaiser, Polte - Partnerschaft mbB
(86) International application number: PCT/JP2017/007812
(87) International publication number: WO 2017/150536

(57) **Abstract**

This invention relates to a peptide comprising the amino acid sequence LIVTQTMKG (SEQ ID NO: 1) at the N-terminus.

SEQUENCE LISTING

<110>  Kyoto University
       KAZUSA DNA RESEARCH INSTITUTE

<120>  Peptide

<130>  P17-048WO

<150>  JP 2016-037673
<151>  2016-02-29

<160>  1

<170>  PatentIn version 3.5

<210>  1
<211>  9
<212>  PRT
<213>  Artificial sequence

<220>
<223>  Designed peptide

<400>  1

Leu Ile Val Thr Gln Thr Met Lys Gly
1               5

## Description

### Technical Field

The present invention relates to a novel peptide.

### Background Art

Ghrelin is a gastrointestinal hormone having an effect of stimulating appetite and an effect of promoting growth hormone secretion. Inhibiting ghrelin secretion is expected to be a means for controlling overeating and obesity, which have recently become a social problem; however, there is no report on the means for inhibiting ghrelin secretion.

Patent Literature 1 discloses an appetite suppressing peptide that promotes the secretion of cholecystokinin (CCK), which is a gastrointestinal hormone having an appetite suppression effect. However, a peptide capable of inhibiting ghrelin secretion to suppress appetite is not known.

### Citation List

### Patent Literature

PTL 1: PCT/JP2011/076862

### Summary of Invention

### Technical Problem

An object of the present invention is to provide a novel peptide having an effect of inhibiting ghrelin secretion, and a pharmaceutical and a food product comprising the peptide.

### Solution to Problem

The present inventors conducted extensive research to solve the above problem. As a result, they found a peptide having an effect of inhibiting ghrelin secretion in a thermolysin digest of β-lactoglobulin protein. The present inventors conducted further research based on this finding and accomplished the present invention.

Specifically, the present invention includes the following embodiments.
Item 1. A peptide comprising the amino acid sequence LIVTQTMKG (SEQ ID NO: 1) at the N-terminus.
Item 2. The peptide according to Item 1, which is derived from a thermolysin digest of milk β-lactoglobulin protein.
Item 3. A pharmaceutical composition comprising the peptide according to Item 1 or 2 as an active ingredient.
Item 4. A ghrelin secretion inhibitor comprising the peptide according to Item 1 or 2 as an active ingredient.
Item 5. A food product comprising the peptide according to Item 1 or 2.
Item 6. A food product characterized in that the peptide according to Item 1 or 2 is added to the food product.
Item 7. The food product according to Item 5 or 6 for suppressing appetite.
Item 8. A method for suppressing appetite, comprising administering the peptide according to Item 1 or 2 to a patient requiring the peptide or a potential patient.
Item 9. The peptide according to Item 1 or 2 for suppressing appetite.
Item 10. Use of the peptide according to Item 1 or 2 for producing a pharmaceutical or a food product for suppressing appetite.

### Advantageous Effects of Invention

A pharmaceutical composition or food product comprising the peptide of the present invention as an active ingredient exhibits an appetite suppression effect based on the effect of inhibiting ghrelin secretion. The pharmaceutical composition or food product of the present invention is suitably used for preventing or treating overeating and obesity based on these effects. The peptide of the present invention has low side effects, and thus is suitable for long-term use. The pharmaceutical composition and food product of the present invention is effective when orally administered.

Since the peptide of the present invention is an enzymatic digest of β-lactoglobulin protein, the side effects should not be a problem. Further, since β-lactoglobulin protein is contained in a large amount in milk, it can be produced at low cost.

### Brief Description of Drawings

Fig. 1 shows effects of enzymatic digests on ghrelin secretion.
Fig. 2 shows effects of peptides on ghrelin secretion.
Fig. 3 shows the results of examining the mechanism of action of ghrelin secretion promotion (cAMP level).
Fig. 4 shows the results of examining the mechanism of action of ghrelin secretion promotion (intracellular calcium ion level) .
Fig. 5 shows effects of oral administration on the serum ghrelin level.

### Description of Embodiments

The peptide of the present invention is a peptide having the amino sequence of 9 residues, i.e., LIVTQTMKG (SEQ ID No: 1), at the N-terminus.

The peptide of the present invention preferably has 9 to 50 amino acid residues, more preferably 9 to 25 amino acid residues, even more preferably 9 to 15 amino acid residues, and particularly preferably 9, 10, 11, 12 or 13 amino acid residues. Of the amino acid sequence shown in SEQ ID No: 1, a peptide consisting of the amino acid sequence at positions 1 to 8, a peptide consisting of the amino acid sequence at positions 1 to 7, a peptide consisting of the amino acid sequence at positions 1 to 6, a peptide consisting of the amino acid sequence at positions 1 to 5, a peptide consisting of the amino acid sequence at positions 1 to 4, and a peptide consisting of the amino acid sequence at positions 1 to 3 are also included as other examples of the present invention.

Each of the amino acids forming the peptide may be an L-amino acid, a D-amino acid, or a DL-amino acid (the mixture of a D-amino acid and an L-amino acid, including both a racemic amino acid and an amino acid containing an excess of either one of the enantiomers). Preferably, the peptide contains only L-amino acids or only D-amino acids. Particularly, a peptide containing only L-amino acids is preferred.

When the peptide used in the present invention has two or more asymmetric carbons, its enantiomer, diastereomer(s), or a mixture thereof in any ratio may also be used. Separation of the enantiomer or diastereomer may be performed by a method using a general column; a method using a chiral column; a method in which the enantiomer or diastereomer is optically resolved in the form of a derivative by introducing an optically active group, and the optically active group is subsequently removed; a method in which the enantiomer or diastereomer is optically resolved by forming a salt with an optically active acid or base; or any other known method.

The peptide may have one or more modifications. The amino terminus (N-terminus) of the peptide may have a modification, such as a free amino group (NH₂-) or an acetyl group (CH₃CO-). The carboxy terminus (C-terminus) of the peptide may have a modification, such as a free carboxy group (-COOH) or an amide group. The amino acid residues of the peptide may be unmodified or have a modification, such as a phosphate group or a sugar chain.

The peptide of the present invention may be in the form of a salt (acid addition salt or base salt). Examples of acid addition salts include salts with inorganic acids, such as hydrochloric acid, sulfuric acid, nitric acid, phosphoric acid, hydrobromic acid, and perchloric acid; and salts with organic acids, such as citric acid, succinic acid, maleic acid, fumaric acid, malic acid, tartaric acid, p-toluenesulfonic acid, benzenesulfonic acid, methanesulfonic acid, and trifluoroacetic acid. Examples of base salts include salts with alkali metals, such as sodium, potassium, and lithium; salts with alkaline earth metals, such as calcium and magnesium; and the like.

The peptide of the present invention may be in the form of a solvate. Examples of solvates include solvates with water (hydrates), methanol, ethanol, isopropanol, acetic acid, tetrahydrofuran, acetone, dimethylformamide, dimethyl sulfoxide, dimethylacetamide, acetamide, ethylene glycol, propylene glycol, dimethoxyethane, and the like.

The peptide of the present invention can be obtained by hydrolyzing a native protein (particularly β-lactoglobulin) or polypeptide, or obtained by chemical synthesis.

For example, the peptide of the present invention can be obtained by hydrolyzing β-lactoglobulin, which is contained in a large amount in whey protein derived from milk, using thermolysin.

Thermolysin is a known proteolytic enzyme (protease) derived from thermostable bacterium Bacillus thermoproteolyticus (EC3.4.24.4). Thermolysin can be used as a food additive in Japan. As thermolysin, commercially available thermolysin of, for example, reagent grade or food additive grade may be used.

The substrate to be hydrolyzed with thermolysin is not particularly limited as long as it contains β-lactoglobulin protein. Examples include milk itself, lactoserum (whey), purified β-lactoglobulin protein, and the like.

Hydrolysis using thermolysin is performed under conditions such that the peptide of the present invention is obtained. The reaction temperature can suitably be selected from, for example, 30 to 70°C, 30 to 40°C, 40 to 70°C, or 50 to 65°C. The reaction time can be suitably selected from, for example, about 30 minutes to 48 hours, about 1 to 10 hours, or about 2 to 8 hours. The pH at which the reaction is carried out can be suitably selected from the pH range of about 6.5 to 8.5 or about 7 to 8. In one preferred embodiment, the reaction can be performed for about 2 to 8 hours under the following conditions: a temperature of about 30 to 40°C and a pH of 6.5 to 8.5 (in particular, about pH 7.5).

The peptide of the present invention may not be obtained under conditions such that hydrolysis is excessively performed.

If necessary, trypsin is deactivated by heating at a temperature at which trypsin is deactivated (for example, heating at a temperature exceeding 80°C for about 5 to 60 minutes).

The hydrolysis product may be used as it is, or purified to separate the active ingredient peptide that is to be used.

The peptide of the present invention can also be obtained by a peptide synthesis method. Specifically, a solution-phase method or a solid-phase method is generally used in peptide synthesis. Among these, a starting material having a reactive carboxy group can be condensed with a starting material having a reactive amino group by a method via an active ester using HBTU etc. or by a method using a coupling agent, such as carbodiimide. When the resulting condensation product has a protecting group, the peptide can also be produced by removing the protecting group.

Functional groups that should not be involved in the reaction of this reaction step are protected with protecting groups. Examples of amino-protecting groups include benzyloxycarbonyl (CBZ), t-butyloxycarbonyl (Boc), 9-fluorenylmethyloxycarbonyl (Fmoc), and the like. Examples of carboxy-protecting groups include groups capable of forming alkyl esters, benzyl esters, and the like. In the case of a solid-phase method, the C-terminal carboxy group is bonded to a support, such as chlorotrityl resin, chloromethyl resin, oxymethyl resin, or p-alkoxybenzyl alcohol resin. The condensation reaction is carried out in the presence of a condensing agent, such as carbodiimide, or using an N-protecting amino acid active ester or a peptide active ester.

The protecting group is removed after the completion of the condensation reaction. In the case of a solid-phase method, the bond between the C-terminus of the peptide and the resin is also cleaved. Furthermore, the peptide of the present invention is purified according to a general method. Examples of purification methods include ion-exchange chromatography, reverse-phase liquid chromatography, affinity chromatography, and the like. The synthesized peptide is analyzed by the Edman degradation technique, using a protein sequencer, GC-MS, or the like that reads an amino acid sequence from the C-terminus.

The peptide of the present invention can also be synthesized according to an enzymatic method (see WO2003/010307).

The peptide of the present invention has an effect of inhibiting ghrelin secretion. Ghrelin is a peptide having the following structure and derived from a living body. Specifically, ghrelin consists of 28 amino acid residues, wherein the serine residue at 3-position is modified with n-octanoic acid. Ghrelin exhibits an effect of stimulating appetite and an effect of promoting growth hormone secretion.

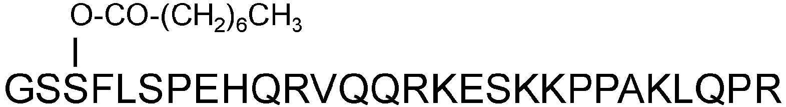

Accordingly, the peptide of the present invention that inhibits ghrelin secretion also has an effect based on ghrelin secretion inhibition, such as an appetite suppression effect. The peptide of the present invention can be used for preventing or treating overeating, obesity, etc., based on the appetite suppression effect.

Those skilled in the art can use a known method to evaluate that the peptide of the present invention promotes the ghrelin output. For example, as described in Iwakura H et al., Endocrinology. 2010 Jun; 151 (6): 2940-5, the ghrelin output can be measured by adding a test substance to MGN3-1 ghrelin secretion cells, and quantifying ghrelin in the medium collected after culturing for a certain period. Ghrelin can be quantified using an immunochemical technique, such as ELISA (Enzyme-Linked Immuno Sorbent Assay).

The peptide of the present invention can be provided as a pharmaceutical composition or a food product (food composition).

The route of administration of the peptide of the present invention or a product containing the peptide of the present invention is not particularly limited. The peptide or the product can be administered orally, parenterally, or intrarectally. The peptide or the product can be administered orally or non-orally. Among these, oral administration is preferable because the obtained effect is high.

The dose of the peptide varies depending on the mode of administration, and the condition, age, and the like of an individual to whom the peptide is administered. The daily dose for an adult is typically 0.01 to 500 mg/kg, preferably 0.05 to 100 mg/kg, and more preferably 0.1 to 30 mg/kg. The peptide (active ingredient) of the present invention is administered in the form of a pharmaceutical composition prepared by mixing with one or more pharmaceutical carriers. A pharmaceutical carrier that is commonly used in the field of pharmaceutical preparations and that does not react with the peptide of the present invention is used.

The peptide of the present invention can be used by itself as a pharmaceutical or a food product, or can be used singly or in a combination with suitable nontoxic carriers for oral administration, diluents, or excipients to make food preparations or pharmaceutical preparations such as tablets (uncoated tablets, sugar-coated tablets, effervescent tablets, film-coated tablets, chewable tablets, and the like), capsules, troches, powders, fine granules, granules, solutions, suspensions, emulsions, pastes, creams, injections (including infusions such as amino acid infusions and electrolyte infusions), and sustained-release preparations including enteric-coated tablets, capsules, and granules. The amount of the peptide in the food product can be suitably selected and is typically in the range of 0.01 to 100 wt%.

Specific examples of pharmaceutical carriers or carriers for oral administration, diluents, excipients, and like substances that can be added to the pharmaceutical or the food product include lactose, glucose, mannite, dextrin, cyclodextrin, starch, sucrose, magnesium aluminometasilicate, synthetic aluminum silicate, sodium carboxymethyl cellulose, hydroxypropyl starch, calcium carboxymethyl cellulose, ion exchange resins, methylcellulose, gelatin, gum arabic, hydroxypropylcellulose, hydroxypropylmethylcellulose, polyvinylpyrrolidone, polyvinyl alcohol, light anhydrous silicic acid, magnesium stearate, talc, tragacanth, bentonite, veegum, titanium oxide, sorbitan fatty acid esters, sodium lauryl sulfate, glycerin, fatty acid glycerol esters, purified lanolin, glycerogelatin, polysorbate, macrogol, vegetable oils, waxes, liquid paraffin, white petrolatum, fluorocarbon, nonionic surfactants, propylene glycol, water, and the like.

Examples of the dosage form include tablets, capsules, granules, powders, syrups, suspensions, suppositories, ointments, creams, gels, patches, inhalants, injections, and the like. These preparations are prepared according to general methods. Liquid preparations may be dissolved or suspended in water or other suitable solvents at the time of use. Tablets and granules may be coated using known methods. Injections are prepared by dissolving the peptide of the present invention in water. As required, injections may also be prepared by dissolving the peptide in physiological saline or a glucose solution, or may additionally contain buffers or preservatives.

These preparations may contain the peptide of the present invention in an amount of 0.01 to 100 wt%, and preferably 1 to 90 wt%. These preparations may also contain other therapeutically beneficial ingredients.

Solid preparations for oral administration may be prepared by mixing the active ingredient with excipients, such as lactose, starch, crystalline cellulose, calcium lactate, and silicic acid anhydride, to form powders. Further, if necessary, for example, binders, such as saccharose, hydroxypropylcellulose, and polyvinylpyrrolidone; and disintegrators, such as carboxymethyl cellulose and calcium carboxymethyl cellulose, may also be added and the resulting mixtures are dry- or wet-granulated to form granules. Tablets may be prepared by tableting these powders or granules as they are, or after adding lubricants, such as magnesium stearate and talc, thereto. These granules or tablets can be coated with enteric coating bases, such as hydroxypropylmethylcellulose phthalate and methacrylic acid-methyl methacrylate polymer, to form enteric-coated preparations; or coated with, for example, ethylcellulose, carnauba wax, or hydrogenated oil to form sustained-release preparations. Capsules may be prepared by filling hard capsules with powders or granules, or by coating with gelatin films the active ingredient as it is, or after being dissolved in glycerin, polyethylene glycol, sesame oil, olive oil, or the like to form soft capsules.

Liquid preparations for oral administration may be prepared by dissolving in water the active ingredient together with sweetening agents, such as saccharose, sorbitol, and glycerin, to form transparent syrups; by further adding essential oils, ethanol, etc., thereto to form elixirs; or by further adding gum arabic, tragacanth, polysorbate 80, sodium carboxymethyl cellulose, etc., thereto to form emulsions or suspensions. These liquid preparations may optionally contain taste-improving agents, coloring agents, preservatives, etc.

Specific examples of forms of food products that can be prepared by adding or blending the peptide of the present invention include beverages (such as coffee, cocoa, juices, soft drinks, mineral drinks, tea beverages, green tea, black tea, oolong tea, milk beverages, lactic acid bacteria beverages, yoghurt beverages, carbonated beverages, and like nonalcoholic drinks, and alcoholic drinks), confectioneries (such as hard candies, gum, gummi candies, jellies, pudding, mousses, cakes, candies, cookies, crackers, biscuits, chocolate, and ice confectioneries (for example, ice creams, sherbets, and shaved ice)), seasoned powder to be sprinkled over rice, dressings, seasonings, processed-soybean products (for example, *tofu* (bean curd), miso, soy sauce, *yuba* (bean curd sheet), soybean flour, and fermented soybeans), processed meat products (for example, hamburger steaks, meat loaf, meatballs, and *tsukune* (meat formed into bite-sized balls)), processed fish meat products (for example, *kamaboko* (steamed fish paste) and *chikuwa* (a kind of fish paste)), retort foods, jelly-like foods (for example, jellies, agar, and jelly-like beverages), and the like. Food products that can be prepared by adding or blending the peptide of the present invention may take the form of health foods, functional foods, foods with function claims, nutritional supplements, dietary supplements, foods for specified health uses, foods for the ill/combined foods for the ill (a category of foods for special dietary uses, approved by Japan's Ministry of Health, Labour and Welfare), and foods for the elderly (a category of foods for special dietary uses, approved by Japan's Ministry of Health, Labour and Welfare). These foods may be in the form of uncoated tablets, film-coated tablets, sugar-coated tablets, granules, powders, tablets, capsules (including both hard and soft capsules), chewable forms, syrups, drinks, and the like. The preparation of food products obtained by adding or blending the peptide of the present invention can be performed according to known methods.

### Examples

The present invention is described in more detail below with reference to Examples. The Examples, however, do not limit the scope of the invention.

### Method

### Evaluation of Ghrelin Secretion Activity

MGN3-1 ghrelin secretion cells were inoculated in a 96-well plate in an amount of 1 x 10⁵ cells per well, and cultured in a medium for 24 hours. After culturing, the cells were washed with DPBS, followed by the addition of 100 µL of a test substance (Buffer: 50 µM sodium octanoate/DMEM). A buffer alone containing no test substance was used as a control. After culturing for another 4 hours, the medium was collected, and a supernatant was obtained by centrifugation. 10 µL of 1N HCl was added to the supernatant, followed by storing at -80°C.

The ghrelin level in the sample was evaluated by ELISA (produced by Bertin Pharma, Ghrelin (Acylated) EIA Kit A05117).

### Production Example

### Enzymatic Digest

Purified milk β-lactoglobulin (β-LG) protein and a digestive enzyme were mixed at the weight ratio of enzyme : β-LG = 1 : 100 (final concentration of β-LG: 10 mg/mL), and a reaction was carried out in the attached buffer.

The enzyme used and reaction conditions were as follows:
(i) thermolysin (Amano Enzyme Inc.); reaction temperature: 37°C, reaction time: 5 hours; reaction buffer: pH 7.5
   After the reaction time above, the sample was boiled (100°C, for 10 minutes) to stop the enzymatic reaction.

### Peptide

Peptide LIVTQTMKG (SEQ ID NO: 1) was synthesized by a standard method.

### Statistical Analysis

The data obtained from the test were represented as the sum of the mean of the number of trials (n) and the standard error of the mean (SEM). The data were analyzed by one-way or two-way ANOVA, followed by the Tukey-Kramer test for multiple comparisons. The difference was considered significant when p<0.05 ("*" in the graphs) or when p<0.01 ("**" in the graphs).

### Test Example

### Test Example 1: β-LG Thermolysin Digest

Using MGN3-1 ghrelin secretion cells, the effect of a β-LG thermolysin digest (β-LG digest) on ghrelin secretion was evaluated.

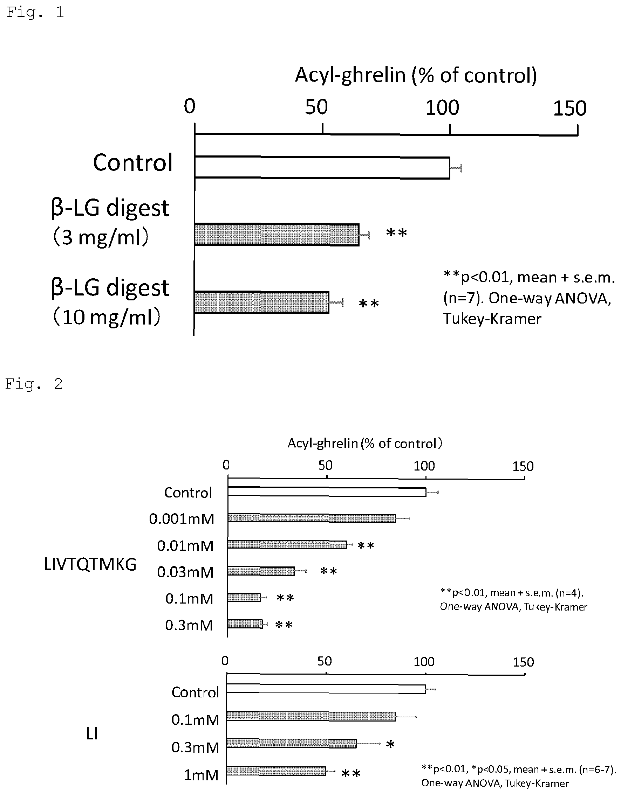

Fig. 1 shows the results (n = 7). In the figure, the horizontal axis "Acyl-ghrelin" (% of control)" indicates the percentage (mass percentage) to the value of the ghrelin output measured when the control (buffer alone containing no test substance) was added to MGN3-1.

The results revealed that the β-LG thermolysin digest (β-LG digest) had an activity of reducing ghrelin output, specifically, an activity of inhibiting ghrelin secretion.

### Test Example 2: Peptide

The concentration dependence of peptide LIVTQTMKG (SEQ ID No: 1) on the effect of inhibiting ghrelin secretion was examined. Dipeptide LI (Comparative Example), which is two residues at the N-terminus, was also evaluated for the effect of inhibiting ghrelin secretion.

Fig. 2 shows the results (n = 4). The results revealed that the peptide LIVTQTMKG showed a significant effect of inhibiting ghrelin secretion at a concentration of 0.01 mM or more. On the other hand, although the dipeptide LI also had an effect of inhibiting ghrelin secretion, it showed a significant effect only at a concentration of 0.3 mM or more.

### Reference Test Example

It was confirmed by mass spectrometry that the peptide LIVTQTMKG (SEQ ID No. 1) and the dipeptide LI used in Test Example 2 were contained in the β-LG thermolysin digest. Table 1 shows the results.

**Table 1**

| Peptide | Formation rate (Mol%) |
|---|---|
| LIVTQTMKG | 27.9 |
| LI | 9.7 |

### Test Example 3: Mechanism of Action

The mechanism of action leading to ghrelin secretion inhibition was examined. It was evaluated how the β-LG thermolysin digest, peptide LIVTQTMKG (SEQ ID NO: 1), and dipeptide LI affected the Forskolin dependent cAMP level elevation and the intracellular calcium ion level ([Ca²⁺]).

In order to measure the intracellular cAMP level, MGN3-1 ghrelin secretion cells were inoculated in a 96-well plate in an amount of 1 x10⁵ cells per well, and cultured in a medium for 24 hours. After the culturing, the cells were washed with DPBS, followed by the addition of 45 µL of a test substance (Buffer: Krebs Ringer HEPES buffer containing 0.5 mM IBMX and 0.01 mM Forskolin). After culturing for another 30 minutes, the intracellular cAMP was quantified by using a Hit Hunter cAMP Assay for small molecules (by DiscoveRx).

The intracellular calcium ion level was measured according to the method described in Kagebayashi T et al., Mol. Nutr. Food Res. 2012 Sep; 56(9): 1456-63.

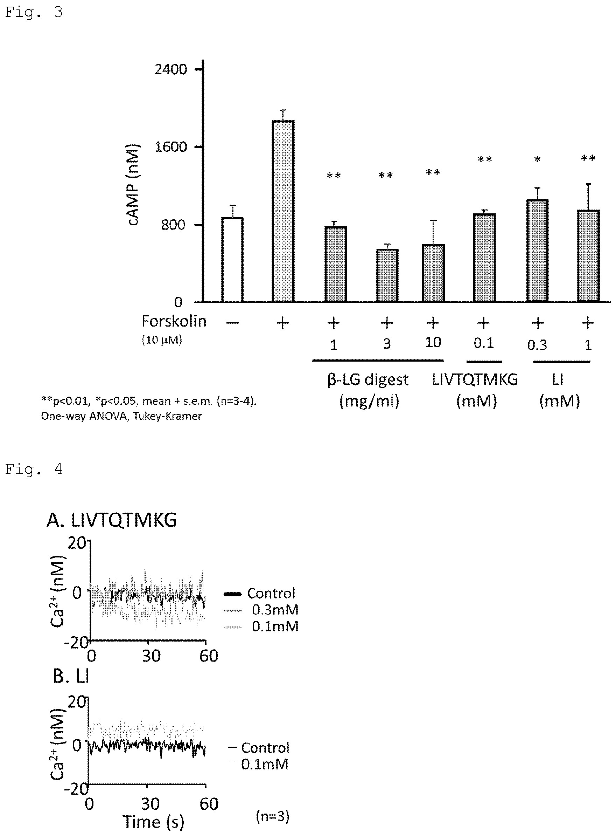

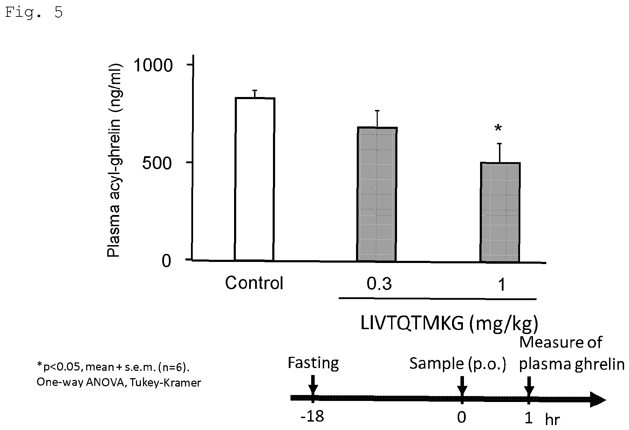

Figs. 3 and 4 show the results. Each of the peptide LIVTQTMKG (SEQ ID No: 1) and the dipeptide LI inhibited the Forskolin dependent cAMP elevation (Fig. 4). Further, the peptide LIVTQTMKG (SEQ ID No: 1) and the dipeptide LI decreased the intracellular calcium level (Fig. 5). From the above results, it is considered that the peptide derived from milk β-LG of the present invention inhibits ghrelin secretion via the conventionally known pathway.

### Test Example 4: Administration In Vivo

Peptide LIVTQTMKG (SEQ ID: No. 1) was administered to a mouse (ddY mouse, male, 34 to 40 g), and the effect on the serum ghrelin level was evaluated. Specifically, peptide LIVTQTMKG (SEQ ID No: 1) that had been dissolved in physiological saline was orally administered (p.o.) in an amount of 0.3 mg/kg or 1 mg/kg to a mouse that had been fasting from 18 hours before administration. One hour later, the serum ghrelin level was measured (measure of plasma ghrelin).

Fig. 5 shows the results (n = 6). The results revealed that the oral administration of peptide LIVTQTMKG reduced the serum ghrelin level of the mouse.

## Claims

1. A peptide comprising the amino acid sequence LIVTQTMKG (SEQ ID NO: 1) at the N-terminus.

2. The peptide according to claim 1, which is derived from a thermolysin digest of milk β-lactoglobulin protein.

3. A pharmaceutical composition comprising the peptide according to claim 1 or 2 as an active ingredient.

4. A ghrelin secretion inhibitor comprising the peptide according to claim 1 or 2 as an active ingredient.

5. A food product comprising the peptide according to claim 1 or 2.

6. A food product **characterized in that** the peptide according to claim 1 or 2 is added to the food product.

7. The food product according to claim 5 or 6 for suppressing appetite.

8. A method for suppressing appetite, comprising administering the peptide according to claim 1 or 2 to a patient requiring the peptide or a potential patient.

9. The peptide according to claim 1 or 2 for suppressing appetite.

10. Use of the peptide according to claim 1 or 2 for producing a pharmaceutical or a food product for suppressing appetite.
